(19) Europäisches Patentamt
European Patent Office
Office européen des brevets

(11) **EP 2 937 424 B1**

(12) **EUROPEAN PATENT SPECIFICATION**

(45) Date of publication and mention of the grant of the patent:
**29.03.2017 Bulletin 2017/13**

(51) Int Cl.:
*C12R 1/25* *(2006.01)*  *A61K 35/747* *(2015.01)*
*C12N 1/20* *(2006.01)*  *A61K 39/00* *(2006.01)*

(21) Application number: **15164896.1**

(22) Date of filing: **23.04.2015**

(54) **LACTIC ACID BACTERIUM, COMPOSITION CONTAINING THE SAME AND THEIR USE**

MILCHSÄUREBAKTERIUM, ZUSAMMENSETZUNG DAMIT UND VERWENDUNG DAVON

BACTÉRIE D'ACIDE LACTIQUE, COMPOSITION LA CONTENANT ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2014 US 201414259442**
**20.04.2015 US 201514690502**

(43) Date of publication of application:
**28.10.2015 Bulletin 2015/44**

(73) Proprietor: **National Yang-Ming University Taipei 112 (TW)**

(72) Inventors:
• **Tsai, Ying-Chieh**
**112 Taipei (TW)**
• **Liu, Wei-Hsien**
**112 Taipei (TW)**
• **Liao, Jian-Fu**
**112 Taipei (TW)**
• **Hsu, Chih-Chieh**
**112 Taipei (TW)**

(74) Representative: **Potter Clarkson LLP**
**The Belgrave Centre**
**Talbot Street**
**Nottingham NG1 5GG (GB)**

(56) References cited:
• **NOBAEK S ET AL: "ALTERATION OF INTESTINAL MICROFLORA IS ASSOCIATED WITH REDUCTION IN ABDOMINAL BLOATING AND PAIN IN PATIENTS WITH IRRITABLE BOWEL SYNDROME", AMERICAN JOURNAL OF GASTROENTEROLOGY, ELSEVIER SCIENCE INC, US, vol. 95, no. 5, 1 May 2000 (2000-05-01), pages 1231-1238, XP009042093, ISSN: 0002-9270, DOI: 10.1111/J.1572-0241.2000.02015.X**
• **WEI-HSIEN LIU ET AL: "Genome architecture of Lactobacillus plantarum PS128, a probiotic strain with potential immunomodulatory activity", GUT PATHOG, vol. 7, 1 January 2015 (2015-01-01), page 22, XP055211341, DOI: 10.1186/s13099-015-0068-y**

EP 2 937 424 B1

SEQUENCE LISTING

<110> National Yang-Ming University

<120> LACTIC ACID BACTERIUM, COMPOSITION CONTAINING THE SAME AND THEIR USE

<130> LOUBP/P56511EP

<150> US 14/259442
<151> 23 April 2014

<150> US 14/690502
<151> 20 April 2015

<160> 2

<170> PatentIn version 3.5

<210> 1
<211> 22
<212> DNA
<213> Artificial Sequence

<220>
<223> primer for ERIC-PCR

<220>
<221> misc_difference
<222> (1)..(22)

<400> 1
atgtaagctc ctggggattc ac        22

<210> 2
<211> 22
<212> DNA
<213> Artificial sequence

<220>
<223> primer for ERIC-PCR

<220>
<221> misc_difference
<222> (1)..(22)

<400> 2
aagtaagtga ctggggtgag cg        22

**Description**

BACKGROUND OF THE INVENTION

1. Field of the Invention

This invention relates to a lactic acid bacterium, and more particularly relates to a novel lactic acid bacterium strain for prophylaxis or treatment of a stress-induced disorder or a functional gastrointestinal disorder in a subject.

2. Description of Related Art

Functional gastrointestinal disorders (FGIDs) are the most common problem in gastroenterological practice. These occur as a result of abnormal functioning of the GI tract, and they are defined by chronic abdominal symptom complexes, such as abdominal pain, diarrhea, constipation, and bloating. According to Rome III criteria, more than 20 functional GI disorders have been identified. Common FGIDs include, but are not limited to functional abdominal pain, irritable bowel syndrome (IBS), constipation, functional diarrhea and functional dyspepsia.

Irritable bowel syndrome is a chronic functional gastrointestinal disorder. About 4-30% people worldwide suffer from IBS. IBS has been characterized by two major symptoms: abdominal pain (chronic pain) and bowel habits alteration. According to the predominant symptom, IBS can be classified into four subgroups: IBS with diarrhea (IBS-D), IBS with constipation (IBS-C), mixed IBS (IBS-M), and un-subtyped IBS (IBS-U). In IBS patients, about 30% patients suffer from IBS after gastrointestinal inflammation, and those belong to post-infectious/inflammatory IBS. In addition, the major factor in motivating patients to seek healthcare and causing a significant reduction in quality of life is abdominal pain, which is associated with visceral hypersensitivity (VH). Visceral hypersensitivity is considered to be one of the main mechanisms causing functional gastrointestinal disorders. Moreover, recent studies show that visceral hypersensitivity is highly specific for IBS.

Visceral hypersensitivity is defined as the increased intensity of sensations and the lowered thresholds for visceral pain in patients. The persistent VH is associated with neuronal sensitization, which manifests as an increase in neuronal excitability. Neurotransmitters, such as serotonin (5-hydroxtryptamine, 5-HT), play important roles in neuronal sensitization. Serotonin is a monoamine neuron transmitter. Previous study have shown that awake rats with subcutaneous injection of 5-hydroxytryptophan (5-HTP), a precursor of serotonin, induced VH. The predominant site of serotonin synthesis and storage is the enterochromaffin cells of the intestinal mucosa. Serotonin released from enterochromaffin cells activates neural reflexes associated with intestinal secretion, motility, and sensation. According to clinical studies, patients with IBS are usually accompanied with abnormality of serotonin metabolism. Furthermore, serotonin receptor antagonists have been used widely as a therapeutic drug, indicating that the pathology of IBS is associated with serotonin.

Lowered pain threshold in a rectal distention test, representing visceral hypersensitivity, is a hallmark of IBS patients. In animal studies, colorectal pain threshold can be monitored by electromyography (EMG) signals responded to a barostat distension, and a higher response of EMG signal represents a higher sensitivity to pain. In addition to VH, an increased expression of substance P in the spinal cord has also been demonstrated as a biomarker representing visceral pain sensation during colorectal distension in an IBS rat model.

There are various therapeutic methods to ameliorate symptoms of IBS, including as serotonin receptor antagonists, antidepressant drugs, prokinetics drugs, antispasmodics drugs and probiotics. Probiotics are live microorganisms which, when administered in adequate amounts, confer a health benefit on the host. Due to the absence of pharmacological side effects, probiotics appear to be a potential method for the treatment of IBS. Besides, specific probiotics have been described to confer beneficial effects on constipation symptoms.

Constipation, a worldwide functional gastrointestinal disorder, is defined as infrequent or difficult passage of stool. Constipation has many causes, including chemical compounds, dietary habits, intestinal flora composition, pregnancy, and psychological stress. Although many types of purgative drugs have been identified, most of these drugs have potentially adverse side effects such as inducing tolerance, melanosis coli, or cathartic colon. There is a dysbiosis of the intestinal flora in patients with constipation, which could be improved by consumption of probiotics. Furthermore, probiotic bacteria, especially lactobacilli and bifidobacteria, may lower the pH of the colon, producing lactic acid, acetic acid, and others. A lower pH tends to increase colonic peristalsis and, in consequence, decrease colonic transit time, with a beneficial effect in the treatment of constipation symptoms.

Probiotics can make host gain healthy benefit via keeping gut flora balance, strengthening mucosal barrier function and modulating immune system. There are lots of evidences indicating that probiotics can act as a therapeutic way of diseases, including infectious diseases, allergy, cancer and necrotising enterocolitis. It is worth to further research the use of probiotics in treating functional gastrointestinal disorders.

Among these, lactic acid bacteria (hereinafter sometimes referred to as LAB), the most important microbial species that are used as probiotics, have been recognized as alternatives of prevention or treatment for gut health because of their capability in regulating host's gut microbiota. Besides, in recent years, the increased evidences also emerge that LAB are able to alter host's mental and physical responses for psychological stress.

Stress is one of the factors inducing mood disorders and neurochemical changes in both human and animals. Rodents subjected to forced-swimming as an acute stressor often show increased immobility in the re-test on next day. Studies have shown that exposing rodents to early life stress by maternal deprivation leads to multiple abnormalities including intestinal inflammations, anxiety-like and depression-like behaviors in adulthood, impaired function of hypothalamus-pituitary-adrenal (HPA) axis, and altered neurotransmitters compared to their normal-reared controls. Stress-induced disorders include, but are not limited to anxiety, depression and irritable bowel syndrome.

Treatment of stress-induced disorders by LAB might be a supplement except conventional psychiatric medicines as LAB have been shown to have an influence on host gut-brain axis (GBA). It was demonstrated that germ-free mice lack the increased motor activity and reduced anxiety had put the emphasis on the key role of normal gut microbiota in brain development. Previous study showed that *Lactobacillus rhamnosus* altered functions of central nerve system in healthy mice through vagus nerve, and the maternal separated rats with probiotic strain *Bifidobacterium infantis* had reversed the behavioral deficits in forced swimming test (FST) and restored basal noradrenaline (NA) level in brainstem. The above-mentioned studies support that probiotics might be developed as treatment on psychiatric disorders and neurotransmitter.

Many studies had show that production and manufacturing methods and the food carrier may influence the properties of probiotic strains, and have an impact on the outcome of clinical intervention studies. Here, we identified a lactic acid bacterium strain showed the potential that preventing or treating psychiatric disorders and functional gastrointestinal disorders.

## SUMMARY OF THE INVENTION

The present invention provides an isolated lactic acid bacterium, which is *Lactobacillus plantarum* subsp. *plantarum* PS128 (hereinafter sometimes referred to as PS128) and deposited under DSMZ Accession No. DSM 28632.

In one aspect of the present application, a composition that comprises *Lactobacillus plantarum* subsp. *plantarum* PS128 and a carrier is provided.

In one embodiment, the carrier is a physiologically acceptable excipient or diluent. The examples of the physiologically acceptable excipient or diluent include, but are not limited to, lactose, starch, dextrin, cyclodextrin, sodium carboxymethyl starch, carboxylated starch propionate, microcrystalline cellulose, carboxymethyl cellulose, maltodextrin and magnesium stearate.

In one aspect of the present application, a method for preventing or treating a stress-induced disorder or a psychiatric disorder in a subject that comprises a step of administering an effective amount of *Lactobacillus plantarum* subsp. *plantarum* PS128 to the subject is provided. Preferably, the stress-induced disorder or the psychiatric disorder is selected from the group consisting of anxiety, depression and irritable bowel syndrome.

In a further embodiment of the present application, after the administration of *Lactobacillus plantarum* subsp. *plantarum* PS128, a serum corticosterone level is statistically significantly decreased in the subject. Moreover, after the administration of *Lactobacillus plantarum* subsp. *plantarum* PS128, a neurotransmitter level is statistically significantly increased in the subject. Preferably, the neurotransmitter is dopamine.

In one aspect of the present application, a method for preventing or treating a functional gastrointestinal disorder in a subject that comprises a step of administering an effective amount of *Lactobacillus plantarum* subsp. *plantarum* PS 128 to the subject is provided.

In another aspect of the present invention, a method for preventing or treating visceral hypersensitivity in a subject that comprises a step of administering an effective amount of *Lactobacillus plantarum* subsp. *plantarum* PS128 to the subject is provided. Preferably, the visceral hypersensitivity is associated with a functional gastrointestinal disorder.

In one embodiment, the functional gastrointestinal disorder is selected from the group consisting of functional abdominal pain, irritable bowel syndrome, constipation, functional diarrhea and functional dyspepsia. In a preferable embodiment, the functional gastrointestinal disorder is functional abdominal pain, irritable bowel syndrome or constipation.

In another aspect of the present invention, a method for preventing or treating functional abdominal pain, irritable bowel syndrome or constipation in a subject that comprises a step of administering an effective amount of *Lactobacillus plantarum* subsp. *plantarum* PS128 to the subject is provided.

In a further embodiment of the present application, after the administration of *Lactobacillus plantarum* subsp. *plantarum* PS128, a level of a biomarker of pain sensation is statistically significantly decreased in the subject. Preferably, the biomarker is substance P. Moreover, after the administration of *Lactobacillus plantarum* subsp. *plantarum* PS 128, an electromyography signal is statistically significantly decreased in the subject.

In a preferable embodiment, the isolated lactic acid bacterium is orally-administered to the subject.

BRIEF DESCRIPTION OF THE DRAWINGS

FIG. 1 is an electrophoresis photograph showing the ERIC-PCR profiles of *Lactobacillus plantarum* strains, wherein M represents DNA ladder; DSM 27445 represents *Lactobacillus plantarum* subsp. *plantarum;* ATCC 14917[T] represents *Lactobacillus plantarum* subsp. *plantarum;* and ATCC 17638[T] represents *Lactobacillus plantarum* subsp. *argentoratensis.*

FIGs. 2A and 2B show that PS128 increases total distance moved in the open field test and normalizes depression-like behavior in the forced swimming test of the mice (*$p < 0.05$ compared to Control group; $\$p < 0.05$ compared to Stress group).

FIG. 3 shows the concentration of serum corticosterone (*$p < 0.05$ compared to Control group; $\$p < 0.05$ compared to Stress group).

FIGs. 4A to 4C show alterations of PS128 on prefrontal cortical dopaminergic pathway of mice (*$p < 0.05$ compared to Control group; $\$p < 0.05$ compared to Stress group).

FIG. 5A shows an experimental timeline for assessing the effect of PS128 on 5-HTP-induced visceral hypersensitivity and FIG. 5B shows the experimental procedures of visceral hypersensitivity test.

FIGs. 6A to 6C show electromyography (EMG) signals responded to CRD stimulation (n=8, *$p < 0.05$, **$p < 0.01$, ***$p < 0.0001$, baseline vs. 5-HTP).

FIG. 7 shows the western blotting analysis of substance P level in L6-S1 spinal cord, which was isolated from a rat with or without PS 128 administration (n=3, ***$p < 0.0001$).

FIGs. 8A and 8B show the effects of orally administered PS 128 on stool weight in normal (A) and experimental constipated (B) mice (n=6, **$p < 0.01$ , *$p < 0.05$).

DETAILED DESCRIPTION OF THE PREFERRED EMBODIMENTS

The following specific examples are used for illustrating the present invention. A person skilled in the art can easily conceive the other advantages and effects of the present invention. The present invention can also be implemented by different specific cases be enacted or application, the details of the instructions can also be based on different perspectives and applications in various modifications and changes do not depart from the spirit of the creation.

Many examples have been used to illustrate the present invention. The examples sited below should not be taken as a limit to the scope of the invention.

EXAMPLES

EXAMPLE 1. Isolation of *Lactobacillus plantarum* subsp. *plantarum* PS 128 and discrimination of the novel Bacterial Strains Using ERIC-PCR profiles

*Lactobacillus plantarum* subsp. *plantarum* PS128 was isolated from fu-tsai, traditional fermented mustard products of Taiwan.

ERIC-PCR was conducted to further distinguish the subspecies of bacteria with high sequence similarity.

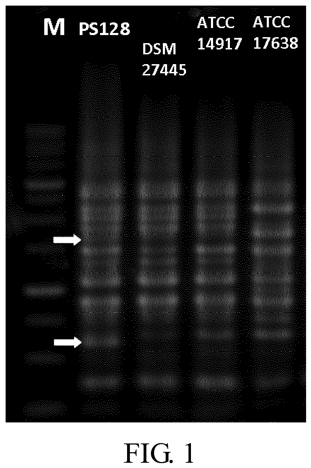

The ERIC-PCR profile of *Lactobacillus plantarum* strains was carried out under the condition indicated in Table 1 and Table 2. DNAs extracted from PS128 and three *Lactobacillus plantarum* strains were used as templates. The obtained amplification products were electrophoresed and the patterns were compared as shown in FIG. 1, wherein the primers represented by SEQ ID NO: 1 and SEQ ID NO: 2 were used.

ERIC1R: (5'- ATGTAAGCTCCTGGGGATTCAC -3') SEQ ID NO: 1
ERIC2: (5'- AAGTAAGTGACTGGGGTGAGCG -3') SEQ ID NO: 2

Table 1. Composition of the PCR reaction solution (25 µl per PCR tube)

| Component | Volume |
|---|---|
| ddH$_2$O | 16.3 µl |
| 10X PCR Buffer | 2.5 µl |
| dNTP | 2.0 µl |

(continued)

| Component | Volume |
|---|---|
| MgCl$_2$ (25 mM) | 1.0 μl |
| primer (GTG)$_5$ (10 μM) | 2.0 μl |
| rTaq polymerase | 0.2 μl |
| DNA template (10μM) | 1.0 μl |

Table 2. PCR Conditions

| Temperature | Time | Cycle |
|---|---|---|
| 94°C | 5 min | |
| 94°C | 30 sec | |
| 45°C | 1 min | 35 cycles |
| 65°C | 6 min | |
| 65°C | 10 min | |

As shown in FIG. 1, Lane M represents DNA ladder (250-10000 bp); DSM 27445 represents *Lactobacillus plantarum* subsp. *plantarum;* ATCC 14917[T] represents *Lactobacillus plantarum* subsp. *plantarum;* and ATCC 17638[T] represents *Lactobacillus plantarum* subsp. *argentoratensis.*

As indicated by white arrows, the bands of PS128 are unique in position among those of DSM 27445, ATCC 14917[T] or ATCC 17638[T] and hence the result in FIG. 1 shows that even though PS 128, DSM 227445 and ATCC 14917[T] all belong to *Lactobacillus plantarum* subsp. *plantarum,* they are still different bacterial strains. Consequently, PS128 represented a new strain of *Lactobacillus plantarum* subsp. *plantarum.*

*Lactobacillus plantarum* subsp. *plantarum* PS 128 has been deposited under Budapest Treaty at Leibniz Institute DSMZ-German Collection of Microorganisms and Cell Cultures (Inhoffenstr. 7 B, D-38124 Braunschweig, Germany) on March 31, 2014 and has been given the DSMZ Accession No. DSM 28632 by the International Depositary Authority. This biological material was subjected to the viability test and passed.

EXAMPLE 2. Analytical Profile Index (API) Typing

Sugar utilization for PS 128 used in the present invention was investigated using API 50 CHL kit (bioMerieux, France), and the results are shown in Table 3. The fermentation test indicates that PS128 harbor a biochemical property similar to *Lactobacillus plantarum* subsp. *plantarum.*

Table 3: Results of Fermentation Test[a]

| Carbohydrates Substrate Strips | PS128 |
|---|---|
| CONTROL | - |
| Glycerol | - |
| Erythritol | - |
| D-Arabinose | - |
| L-Arabinose | + |
| D-Ribose | + |
| D-Xylose | + |
| L-Xylose | - |
| D-Adonitol | - |

(continued)

| Carbohydrates Substrate Strips | PS128 |
|---|---|
| Methyl-β-D-Xylopyranoside | - |
| D-Galactose | + |
| D-Glucose | + |
| D-Fructose | + |
| D-Mannose | + |
| L-Sorbose | - |
| L-Rhamnose | + |
| Dulcitol | - |
| Inositol | - |
| D-Mannitol | + |
| D-Sorbitol | + |
| Methyl-α-D-mannopyranoside | + |
| Methyl-α-D-glucopyranoside | - |
| N-Acetyl glucosamine | + |
| Amygdalin | + |
| Arbutin | + |
| Esculin ferric citrate | + |
| Salicin | + |
| D-Cellobiose | + |
| D-Maltose | + |
| D-Lactose (bovine origin) | + |
| D-Melibiose | + |
| D-Saccharose (sucrose) | + |
| D-Trehalose | + |
| Inulin | + |
| D-Melezitose | + |
| D-Raffinose | + |
| Amidon (starch) | - |
| Glycogen | - |
| Xylitol | - |
| Gentiobiose | + |
| D-Turanose | + |
| D-Lyxose | - |
| D-Tagatose | + |
| D-Fucose | - |
| L-Fucose | - |
| D-Arabitol | - |

(continued)

| Carbohydrates Substrate Strips | PS128 |
| --- | --- |
| L-Arabitol | - |
| Potassium gluconate | + |
| Potassium 2-ketogluconate | - |
| Potassium 5-ketogluconate | - |
| a. +, positive; -, negative; | |

EXAMPLE 3. Alternations of Behaviors, Stress hormone and Neurotransmitter Levels in Stressed Mice by *Lactobacillus plantarum* subsp. *plantarum* PS128

(1) Preparation of PS 128

PS128 was inoculated in culture medium (10% skim milk, 1% yeast powder, 0.1% tween 80, and 2% glucose), cultured at 37°C for 18 hrs and harvested by centrifugation. PS128 was embedded and lyophilized with protective agents (skim milk 1%, Sugar 2%, oligofructose 2%, maltodextrin 3%, and glycerol 2%) and excipients to a final concentration of $1 \times 10^{11}$ colony formation unit (CFU) per gram powder. PS128 powder was stored at -20°C and stored at -20°C until use.

(2) Animals and Housing

Eight-week-old Male C57BL/6JNarl or timed-pregnant C57BL/6JNarl were purchased from National Laboratory Animal Center (NLAC, Taipei, Taiwan) and housed in filter-top cages with chow diet (LabDiet Autoclavable Rodent Diet 5010, PMI Nutrition International, Brentwood, USA) and water *ad libitum* in a specific pathogen-free room at Laboratory Animal Center, National Yang-Ming University under standard condition (temperature 22°C, 50-60% humidity and 12-h light/dark cycle) for approximately one week until delivery. All animal experimental procedures were reviewed and approved by the Animal Management Committee, National Yang-Ming University.

(3) Early Life Stress

Early life stress by maternal deprivation under hypothermal environment was modified from previous studies (Millstein and Holmes 2007, Desbonnet, Garrett et al. 2010). Briefly, neonatal were separated from their mothers and littermates 3h (11:00-14:00) daily between postnatal day (PD) 2-14 under room temperature (~22°C). Followed 2 weeks undisturbed except bedding change once per week, the stressed pups were weaned and randomly assigned to 5-6 mice per cage. Only male pups were used for experiment (n=12 for Stress groups, n=12 for Stress+$10^9$). The mice that did not receive early life stress were served as control group (Control, n=10).

(4) Experimental Design and Sampling

Mice were administrated with saline or PS128 ($10^9$ CFU in 0.2 mL saline/mouse/day) for 4 weeks from weaning (PD28) to the age of 8-weeks-old. They underwent a battery of behavioral tests including OFT and FST sequenced from the least to the most stressful ones conducted in light phase. On the day of sacrifice, mice were subjected to retro-orbital blood collection followed cervical dislocation which all took place between 11:00 and 14:00 h to minimize the effects of circadian rhythm. Brain were quickly removed and specific region prefrontal cortex was dissected on ice and frozen in liquid nitrogen or preserved in 0.6% perchloric acid buffer, then stored at -80°C until use.

(5) Open Field Test (OFT)

The open field test (OFT) is a common measure of exploratory behavior and general activity in both mice and rats, where both the quality and quantity of the activity can be measured.

Locomotor activity was monitored and calculated by an open field activity system (Tru Scan activity system, Coulbourn Instruments, PA, USA) comprised of one arena (25.4 × 25.4 × 38 cm) with two photobeam sensor rings outside of the Plexiglas wall. Each mouse was placed into the same corner of the arena for 10 min to measure several activities including total distance moved. The box was cleaned with water and 70% ethanol after each run, and the mouse was returned to its home cage. The activities were automatically recorded and quantified by the Tru Scan 2.2 software (Coulbourn Instruments).

(6) Forced Swimming Test (FST)

The forced swimming test is a rodent behavioral test used for evaluation of antidepressant drugs, antidepressant efficacy of new compounds, and experimental manipulations that are aimed at rendering or preventing depressive-like states.

FST were assessed as previously described (Cryan, Dalvi et al. 2001) with modifications. Briefly, immobility was calculated 4 min (1-5 min) of a single 6-min forced swimming test which the mice were put in a transparent acrylic cylinder (30 cm height × 10 cm internal diameter, containing 15 cm-depth water with temperature adjusted to 23 ± 1°C), recorded by a camera and further analyzed by a video tracking software EthoVision (Noldus Information Technology, Wageningen, The Netherlands). Mice were dried with tissue paper after tested and returned to their home cage. Immobility was calculated for 1 to 5 min with the same parameters to avoid human errors.

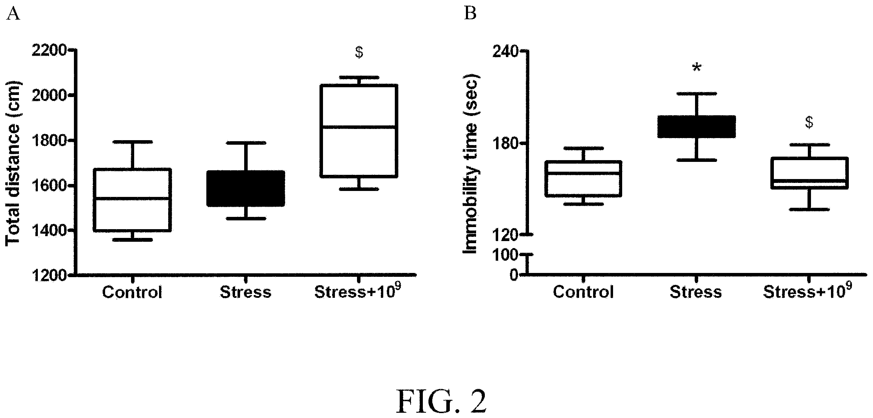

As shown in FIG.s 2A to 2B, in the OFT, the stressed mice had similar total distance moved compared to Control (FIG. 2A) while they had greater time of immobile in FST (FIG. 2B). Administration of PS128 to the stressed mice significantly increased the total distance moved in the OFT and decreased the immobility in the FST (FIGs. 2A and 2B) to the degrees similar to Control mice. *$p < 0.05$ compared to Control group; $$p < 0.05$ compared to Stress group.

(7) Determination of Serum Corticosterone

Plasma corticosterone level is an indicator the activation of the hypothalamic-pituitary-adrenal axis, the basic response in body-brain interaction under stress or fear.

Serum was obtained from blood samples centrifuged at 4°C, 3000×g for 10 min that were collected from mice before sacrifice. Serum was properly diluted and then applied to a commercial CORT EIA kit (Cayman, USA), concentrations were interpolated by standards provided by the kit.

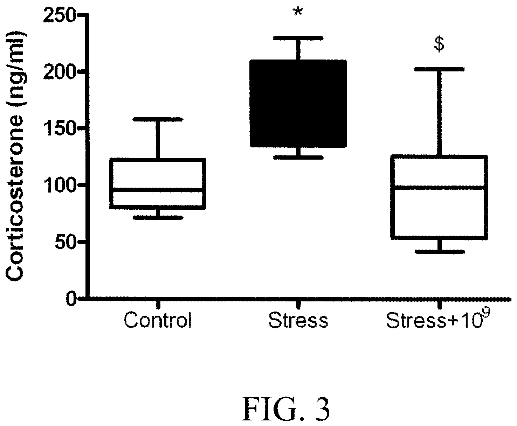

As shown in FIG. 3, by measuring the serum level, the inventors found that corticosterone was significantly elevated in the stressed mice and was reversed by the administration of PS128 (Stress+$10^9$) . *$p < 0.05$ compared to Control group; $$p < 0.05$ compared to Stress group.

(8) Quantification of Monoamines and Their Metabolites by HPLC-ECD

HPLC-ECD has the high performance for routine measurements of biological or environmental samples such as catecholamines (dopamine, norepinephrine, and epinephrine), monoamines (e.g., serotonin, dopamine, norepinephrine, and epinephrine) acetylcholine, glutamate, glycine, GABA, and others.

The neurotransmitters to be measured include dopamine (DA) and its metabolites, dihydroxyphenylacetic acid (DC) and homo-vanillic acid (HVA). The high performance liquid chromatography-electrochemical detector (HPLC-ECD) system comprised a micropump (CMA-100, CMA, Stockhom, Sweden), an on-line injector (CMA-160), a Microtech LC-pump (Micro-tech Scientific, Sunnyvale, CA), and BAS-4C electrochemical detector (Bioanalytical Systems, Inc., West Lafeyette, IN) as previously described (Cheng, Kuo et al. 2000). A reversed-phase column (Kinetex C$_{18}$, 2.6 um, 100 x 2.1 mm I.D., Phenomenex, USA) was used for analysis. The potential for the glassy carbon working electrode was set at +650 mV with respect to a Ag/AgCl reference electrode at room temperature (25°C). The mobile phase containing 0.1 M NaH$_2$PO4, 8% methanol, 0.74 mM SOS, 0.03 mM EDTA and 2 mM KCl, was adjusted to pH 3.74 with H$_3$PO$_4$. Whole brains were frozen on dry ice immediately after collected from cervical dislocated mice. Prefrontal cortex was dissected and preserved in 0.6% perchloric acid buffer and stored at -80°C until homogenized by sonication and centrifuged at 12000×g, 10 min. Supernatants were filtered by 0.22 m PVDF membrane (4 mm syringe filter, Millex-GV, Millipore, USA) before analyze. Properly diluted supernatants were injected (20 μl) into the chromatographic system at a flow rate of 0.2 ml/min. The concentrations of monoamines were interpolated by the following standards: DA, DC and HVA (Sigma-Aldrich, St. Louis, MO, USA) ranged from 1 to 100 ng/ml.

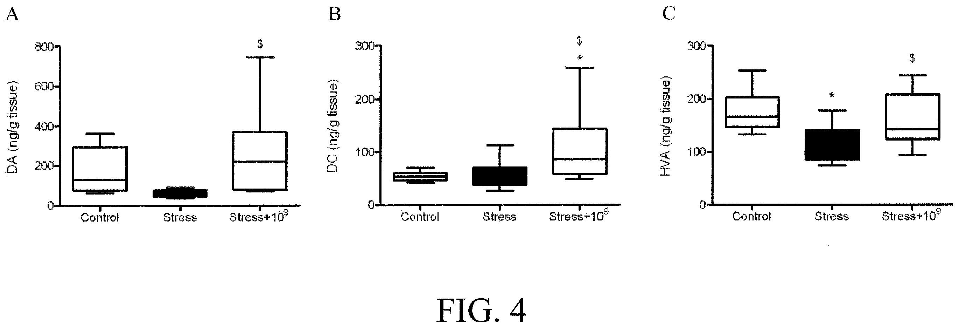

As shown in FIG.s 4A to 4C, by applying the early life stress protocol, neurotransmitters in prefrontal cortex was altered. In the dopaminergic pathway, dopamine itself was reduced and the level of its metabolite DC was similar to that of Control group (FIG.s 4A and 4B) while the other metabolite HVA was significantly decreased (FIG. 4C). Administration of PS128 significantly increased dopamine, DC and HVA levels of the stressed mice (FIGs. 4A to 4C). *$p < 0.05$ compared to Control group; $$p < 0.05$ compared to Stress group.

EXAMPLE 4. Alternations of visceral hypersensitivity and substance P levels in rats by *Lactobacillus plantarum* subsp. *plantarum* PS128

Previous study have shown that awake rats with subcutaneous injection of 5-hydroxytryptophan (5-HTP), a precursor of serotonin, induced VH. In this study, the 5-HTP-induced VH model was used to evaluate ameliorative effects of a probiotic strain, *Lactobacillus plantarum* subsp. *plantarum* PS 128, on rats.

(1) Preparation of PS128

PS 128 was inoculated in culture medium (10% skim milk, 1% yeast powder, 0.1% tween 80, and 2% glucose), cultured at 37°C for 18 hrs and harvested by centrifugation. PS128 was embedded and lyophilized with protective agents (skim milk 1%, Sugar 2%, oligofructose 2%, maltodextrin 3%, and glycerol 2%) and excipients to a final concentration of $1\times10^{11}$ colony formation unit (CFU) per gram powder. PS128 powder was stored at -20°C and was dissolved into $5\times10^9$ CFU/mL in saline solution before animal treatment.

(2) Animals and Housing

Six to eight-week-old Male Sprague-Dawley (SD) rats (220 to 330 g) were purchased from National Laboratory Animal Center (NLAC, Taipei, Taiwan). The rats were housed under constant temperature and humidity with 12-h light-dark cycles, and were given free access to food and water. All animal experimental procedures were reviewed and approved by the Animal Management Committee, National Yang-Ming University.

(3) Experiment Procedure

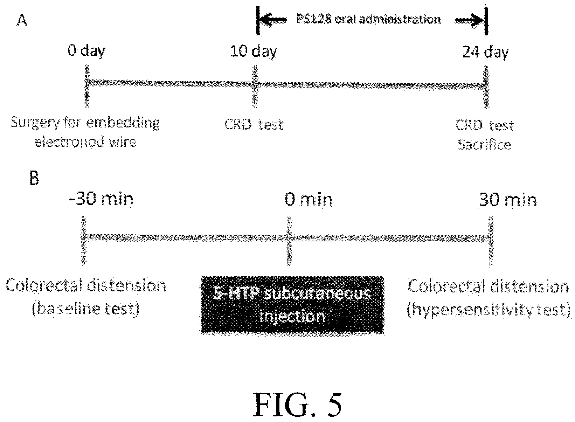

Referring to FIG. 5A, rats at first were anesthetized with isoflurane for electrode implantation. After surgery for embedding electrode, rats were housed in single. On about 7-10 days after the surgery, rats were underwent colorectal distension (CRD) test for baseline and visceral hypersensitivity detection induced by 5-HTP injection. This test was designed to verify the 5-HTP-visceral hypersensitivity in awake male rats without bacteria administration. On day 10, rats were orally administered with PS128 (about $10^9$ CFU in 0.2 mL saline per day) or saline (200 μl per day) for two weeks followed by CRD experiment. After that, another CRD test would be processed to determine the effect of PS128 on VH. The CRD procedure with simultaneous electromyography (EMG) recording was performed. The area under the EMG curve (AUC) was calculated as a parameter. For detecting the protein level of substance P in spinal cord by western blotting, the rats were anesthetized with sodium pentobarbital (65 mg/kg, intraperitoneal injection) and perfused transcardially with normal saline. The L6-S1 spinal cord of rats responsible for abdominal sensation was isolated.

(4) Electrode Implantation

The rats were anesthetic with isoflurane. Electrodes made from Teflon-coated stainless steel wire (A-M systems inc.) were implanted in the rat's abdominal external oblique muscle at least 7 days prior to experimentation. Electrodes were exteriorized onto the back of the neck.

(5) Colorectal distension (CRD) test

Colorectal distension (CRD) is a widely used and reproducible method for assessing visceral sensitivity. The measurement of electromyography signals is one of the evaluation items in CRD and is the most accurate quantitative test item.
Referring to FIG. 5B, CRD tests were processed before and after 5-HTP subcutaneous injection (5 mg/Kg) for determining 5-HTP-induced visceral hypersensitivity.
For the CRD test, rats were placed in plastic tunnels (6-cm diameter, 25-cm length). During 3 days for preceding the test, the rats were trained to the experimental conditions by placing them singly in the tunnel for 3 hours per day. The colorectal distension (CRD) balloon was composed of a latex glove finger (7 cm long) attached to a rectal catheter (Medtronic Inc.). The balloon was inflated and left overnight to help equilibrate the tension in its wall. The inflatable device was introduced through the anal canal completely into the rectum in conscious rats and secured to the tail base. The tube was then connected to a barostat (Medtronic Inc.). The colon was distended by inflating the balloon to the desired pressure (20, 40, or 60 mmHg) for 10-second intervals with 30-second intervals between distensions. Distensions were repeated 4 times for each experimental protocol with 5-minute intervals between each series. The response of CRD was record by an EMG device. The EMG signal analyses were processed by Spike 2 (Cambridge Electronic Design Limited) as visceromotor reflex (area/sec).

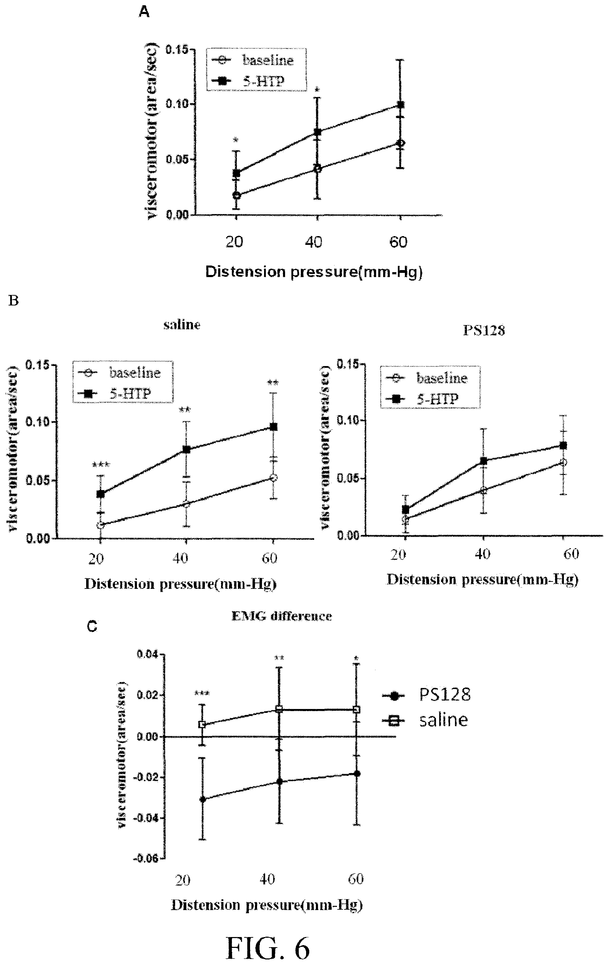

As shown in FIG. 6A, the visceromotor was calculated from the raw EMG signal and increases gradually in a pressure-dependent manner in every groups. Subcutaneous injection of 5-HTP significantly increased the EMG signal in response to CRD relative to the baseline signal in rats.

As shown in FIG. 6B, with two weeks administration of saline, the EMG signals were still elevated significantly after 5-HTP injection while rats with two weeks PS128 administration could inhibit the elevation of EMG signal induced by 5-HTP.

Furthermore, to confirm the effect of administration of PS 128 on inhibiting the elevation of EMG signal, we calculated the difference of the elevation of EMG after 5-HTP injection between day 10 and day 24. The formula of EMG difference is shown below:

$$\text{EMG difference} = (\text{The difference of the elevation of EMG after 5-HTP injection}$$

$$\text{on day 24})-(\text{The difference of the elevation of EMG after 5-HTP injection on day 10})$$

As shown in FIG. 6C, after saline administration, there was more elevation of EMG signal on day 24 compared with that on day 10 in all distension pressure stimulation. In PS128 group, however, there was lower elevation of the EMG signal on day 24 compared with that on day 10 in all distension pressure stimulation. These data indicated that PS 128 had a potential to ameliorate VH induced by 5-HTP injection.

(6) Western blotting

For confirming the effect of PS128 on spinal neuron peptide associated with pain sensation by western blotting, total proteins in the L6-S1 spinal cord tissue were extracted with commercial protein extraction kit (EC21 Inc.). The extractions were fractionated on 10% polyacrylamide gels and then transfer to polyvinylidene difluoride membranes (Roche Ltd.) electrophoretically, followed by blockage for 1 hour with blocking buffer, TBST containing 5% Skim milk, and incubated with the primary antiserum substance P (1:1000; GeneTex inc.) in blocking buffer overnight at 4°C. After twice washed with TBST, membrane was incubated with goat anti-rat IgG-HRP (1:5000; Santa Cruz Biotechnology, inc.) in blocking buffer. The antibody-protein complex was visualized by Immobilon™ Western Chemiluminescent HRP Substrate (Millipore inc.) and detected by Luminescent Image Analyzer (FUJIFILM Holdings Corporation).

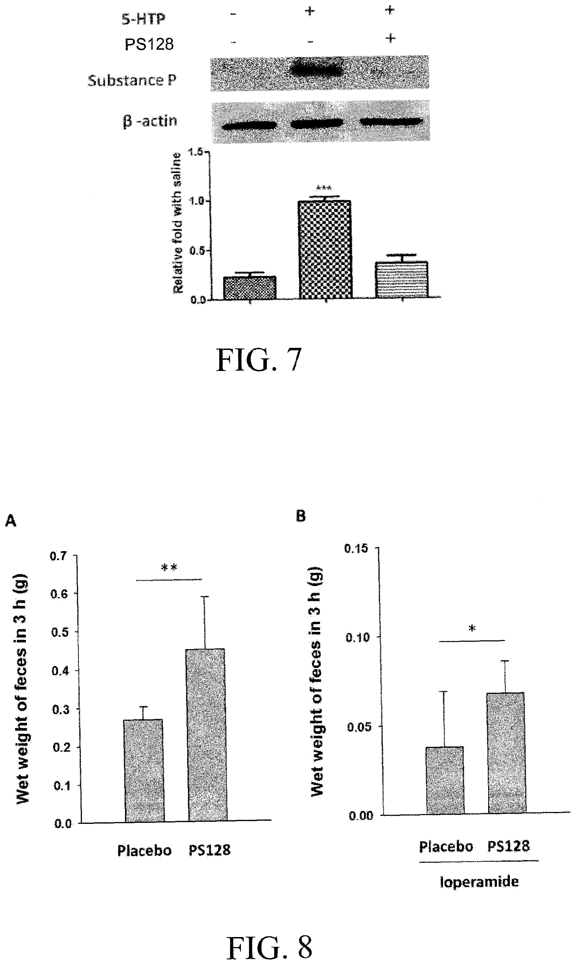

Substance P is a biomarker of pain sensation in the spinal cord of rats. It is suggested that an abnormal expression level of substance P involves in the pathogenesis of IBS. In addition, the substance P-containing neural pathway is considered as one of neural pathways which play a role in the regulation of the gastrointestinal function. As shown in FIG. 7, rats with two weeks saline oral administration were found high level substance P production in L6-S1 spinal cord after 5-HTP injection followed by CRD experiment, while control rats, without 5-HTP injection, had basal level production. Two weeks administration of PS128 inhibited the elevation of substance P production induced by 5-HTP injection. That is, with PS 128 administration, the level of substance P was reversed almost to the level of rats without 5-HTP injection.

EXAMPLE 5. Laxative effect of PS128

Loperamide is an agonist of μ-opioid receptor, which inhibits endogenous acetylcholine release as a result of inhibitions of adenylcyclase via G protein in myenteric plexus. Loperamide is estimated to act directly on the intestinal nerve system to induce constipation and frequently used in constipation model mice.

The laxative effect of PS 128 on evacuating both normal and loperamide-induced constipated mice was further examined. Mice were orally administrated with placebo (0.2 mL saline/mouse/day) or PS128 ($10^9$ CFU in 0.2 mL saline/mouse/day). After 2 weeks, the wet weights of stools from each mouse were measured for 3 hrs. For experimental constipated mice, loperamide hydrochloride (5 mg/kg) was orally administrated, and after 30 minutes, the measurement was initiated and the wet weights of stools from each experimental constipated mouse were measured for 3 hrs. As shown in FIG. 8, the wet weights of stools of PS 128 groups were significantly increased compared with those of the placebo groups, thereby suggesting the laxative effect of PS 128. Those results suggest that PS 128 has the potential of alleviating constipation.

EXAMPLE 6: Statistical Analysis

Differences between experimental groups were analyzed with Prism version 6 (Prism, San Diego, CA). All data presented herein were expressed as means ± the standard deviation (SD). Statistical analysis of Figs. 2, 3, 4, and 7 was calculated by one-way ANOVA followed by a Bonferroni post-hoc test. Two-sample $t$-tests was used to analyze the difference of the EMG signals (Fig. 6) and laxative effect of PS128 (Fig. 8). Differences were considered to be statistically significant if $p<0.05.$ Statistical difference between groups denoted by asterisk (*) or dollar sign ($) if $p<0.05,$ ** if $p<0.01,$ *** if $p<0.0001.$

The present invention finds a potential strain, *Lactobacillus plantarum* subsp. *plantarum* PS128, that exerts benefits on stressed mice and is reported that by administrating a Lactobacillus strain PS128, behaviors of the mice were improved.

In addition, the present invention also finds that PS128 exerts benefits on visceral hypersensitivity and constipation, and PS128 also eliminates the elevation of EMG signals and inhibits the elevated production of substance P in the spinal cord of rats after 5-HTP injection.

Therefore, the method provided by the present invention is useful for the prevention or treatment of a stress-induced disorder or a functional gastrointestinal disorder by administrating a Lactobacillus strain PS128.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claim.

The references listed below and the DSMZ and ATCC numbers cited in the application are each incorporated by reference as if they were incorporated individually.

Chen CL. (2013) "Visceral hypersensitivity in non-erosive reflux disease: neurogenic overwhelming in esophagus?" Dig Dis Sci. Aug;58(8):2131-2.

Collins SM. (2007). "Translating symptoms into mechanisms: functional GI disorders." Adv Physiol Educ. Dec;31 (4):329-31.

Drossman DA. (2006). "The functional gastrointestinal disorders and the Rome III process." Gastroenterology. Apr;130(5):1377-90.

Keohane J, Quigley EM. (2006). "Functional dyspepsia: the role of visceral hypersensitivity in its pathogenesis." World J Gastroenterol. May 7;12(17):2672-6.

Moshiree B, Zhou Q, Price DD, Verne GN. (2006). "Central sensitisation in visceral pain disorders.Gut." Jul;55(7):905-8.

Bouin M, Plourde V, Boivin M, Riberdy M, Lupien F, Laganiere M, Verrier P, Poitras P. Rectal distension testing in patients with irritable bowel syndrome: sensitivity, specificity, and predictive values of pain sensory thresholds. Gastroenterology 2002; 122: 1771-1777.

Chin J Dig Dis. 2006;7(4):211-8. Alternation of substance P-containing neural pathways in a rat model of irritable bowel syndrome with rectal distension.

Neri F, Cavallari G, Tsivian M, Bianchi E, Aldini R, Cevenini M, Guidetti E, Piras GL, Pariali M, Nardo B. J Biomed Biotechnol. 2012;2012:896162. Effect of colic vein ligature in rats with loperamide-induced constipation.

Fausta Serafini, Francesca Turroni, Patricia Ruas-Madiedo, Gabriele Andrea Lugli, Christian Milani, Sabrina Duranti, Nicole Zamboni, Francesca Bottacini, Douwe van Sinderen, Abelardo Margolles, Marco Ventura, Kefir fermented milk and kefiran promote growth of Bifidobacterium bifidum PRL2010 and modulate its gene expression, International Journal of Food Microbiology, 2014, 178, 50-59.

Cheng, F. C., J. S. Kuo, H. M. Huang, D. Y. Yang, T. F. Wu and T. H. Tsai (2000). Determination of catecholamines in pheochromocytoma cell (PC-12) culture medium by microdialysis-microbore liquid chromatography. J Chromatogr A 870(1-2): 405-411.

Cryan, J. F., A. Dalvi, S. H. Jin, B. R. Hirsch, I. Lucki and S. A. Thomas (2001). Use of dopamine-beta-hydroxylase-deficient mice to determine the role of norepinephrine in the mechanism of action of antidepressant drugs. J Pharmacol Exp Ther 298(2): 651-657.

Desbonnet, L., L. Garrett, G. Clarke, B. Kiely, J. F. Cryan and T. G. Dinan (2010). Effects of the probiotic Bifidobacterium infantis in the maternal separation model of depression. Neuroscience 170(4): 1179-1188.

Millstein, R. A. and A. Holmes (2007). Effects of repeated maternal separation on anxiety- and depression-related phenotypes in different mouse strains. Neurosci Biobehav Rev 31(1): 3-17.

## Claims

1. An isolated lactic acid bacterium, being *Lactobacillus plantarum* subsp. *plantarum* PS 128 and deposited under DSMZ Accession No. DSM 28632.

2. A composition comprising the isolated lactic acid bacterium of claim 1, and a carrier.

3. An effective amount of the isolated lactic acid bacterium of claim 1 for use in preventing or treating a stress-induced disorder or a psychiatric disorder in a subject.

4. An effective amount of the isolated lactic acid bacterium of claim 1 for use in a method of claim 3, wherein the stress-induced disorder or the psychiatric disorder is selected from the group consisting of anxiety, depression and irritable bowel syndrome.

5. An effective amount of the isolated lactic acid bacterium of claim 1 for use in a method according to claims 3 and 4

wherein after the administration, a serum corticosterone level is statistically significantly decreased in the subject.

6. An effective amount of the isolated lactic acid bacterium of claim 1 for use in a method according to claims 3 and 4, wherein after the administration, a neurotransmitter level is statistically significantly increased in the subject.

7. An effective amount of the isolated lactic acid bacterium of claim 1 for use in a method according to claim 6, wherein the neurotransmitter is dopamine.

8. An effective amount of the isolated lactic acid bacterium of claim 1 for use in preventing or treating a functional gastrointestinal disorder in a subject.

9. An effective amount of the isolated lactic acid bacterium of claim 1 for use in preventing or treating visceral hypersensitivity in a subject.

10. An effective amount of the isolated lactic acid bacterium of claim 1 for use in a method according to claim 9 wherein the visceral hypersensitivity is associated with a functional gastrointestinal disorder.

11. An effective amount of the isolated lactic acid bacterium of claim 1 for use in a method according to claims 8 to 10, wherein the functional gastrointestinal disorder is selected from the group consisting of functional abdominal pain, irritable bowel syndrome, constipation, functional diarrhea and functional dyspepsia.

12. An effective amount of the isolated lactic acid bacterium of claim 1 for use in preventing or treating functional abdominal pain, irritable bowel syndrome or constipation in a subject.

13. An effective amount of the isolated lactic acid bacterium of claim 1 for use in a method according to claims 8 to 12, wherein after the administration, a level of a biomarker of pain sensation is statistically significantly decreased in the subject, optionally wherein the biomarker is substance P.

14. An effective amount of the isolated lactic acid bacterium of claim 1 for use in a method according to claims 8 to 12, wherein after the administration, an electromyography signal is statistically significantly decreased in the subject.

15. An effective amount of isolated lactic acid bacterium of claim 1 for use in a method according to claims 3 to 14, wherein the isolated lactic acid bacterium is orally-administered to the subject.

**Patentansprüche**

1. Isoliertes Milchsäurebakterium, bei dem es sich um *Lactobacillus plantarum* subsp. *plantarum* PS128 handelt und das bei der DSMZ unter der Zugangsnummer DSM 28632 hinterlegt worden ist.

2. Zusammensetzung, umfassend das isolierte Milchsäurebakterium nach Anspruch 1 und einen Träger.

3. Wirksame Menge des isolierten Milchsäurebakteriums nach Anspruch 1 zur Verwendung beim Vorbeugen oder Behandeln einer stressbedingten Erkrankung oder einer psychischen Erkrankung in einem Subjekt.

4. Wirksame Menge des isolierten Milchsäurebakteriums nach Anspruch 1 zur Verwendung in einem Verfahren nach Anspruch 3, wobei die stressbedingte Erkrankung oder die psychische Erkrankung ausgewählt ist aus der Gruppe bestehend aus Angst, Depression und Reizdarmsyndrom.

5. Wirksame Menge des isolierten Milchsäurebakteriums nach Anspruch 1 zur Verwendung in einem Verfahren nach Anspruch 3 und 4, wobei ein Serum-Corticosteronspiegel in dem Subjekt nach der Verabreichung statistisch signifikant verringert ist.

6. Wirksame Menge des isolierten Milchsäurebakteriums nach Anspruch 1 zur Verwendung in einem Verfahren nach Anspruch 3 und 4, wobei ein Neurotransmitterspiegel in dem Subjekt nach der Verabreichung statistisch signifikant erhöht ist.

**7.** Wirksame Menge des isolierten Milchsäurebakteriums nach Anspruch 1 zur Verwendung in einem Verfahren nach Anspruch 6, wobei der Neurotransmitter Dopamin ist.

**8.** Wirksame Menge des isolierten Milchsäurebakteriums nach Anspruch 1 zur Verwendung beim Vorbeugen oder Behandeln einer funktionellen gastrointestinalen Erkrankung in einem Subjekt.

**9.** Wirksame Menge des isolierten Milchsäurebakteriums nach Anspruch 1 zur Verwendung beim Vorbeugen oder Behandeln einer viszeralen Überempfindlichkeit in einem Subjekt.

**10.** Wirksame Menge des isolierten Milchsäurebakteriums nach Anspruch 1 zur Verwendung in einem Verfahren nach Anspruch 9, wobei die viszerale Überempfindlichkeit mit einer funktionellen gastrointestinalen Erkrankung im Zusammenhang steht.

**11.** Wirksame Menge des isolierten Milchsäurebakteriums nach Anspruch 1 zur Verwendung in einem Verfahren nach Anspruch 8 bis 10, wobei die funktionelle gastrointestinale Erkrankung ausgewählt ist aus der Gruppe bestehend aus funktionellen Bauchschmerzen, Reizdarmsyndrom, Obstipation, funktioneller Diarrhoe und funktioneller Dyspepsie.

**12.** Wirksame Menge des isolierten Milchsäurebakteriums nach Anspruch 1 zur Verwendung beim Vorbeugen oder Behandeln von funktionellen Bauchschmerzen, Reizdarmsyndrom oder Obstipation in einem Subjekt.

**13.** Wirksame Menge des isolierten Milchsäurebakteriums nach Anspruch 1 zur Verwendung in einem Verfahren nach Anspruch 8 bis 12, wobei ein Spiegel eines Biomarkers für Schmerzempfindung in dem Subjekt nach der Verabreichung statistisch signifikant verringert ist, wobei es sich bei dem Biomarker optional um Substanz P handelt.

**14.** Wirksame Menge des isolierten Milchsäurebakteriums nach Anspruch 1 zur Verwendung in einem Verfahren nach Anspruch 8 bis 12, wobei ein Elektromyographiesignal in dem Subjekt nach der Verabreichung statistisch signifikant verringert ist.

**15.** Wirksame Menge von isoliertem Milchsäurebakterium nach Anspruch 1 zur Verwendung in einem Verfahren nach Anspruch 3 bis 14, wobei das isolierte Milchsäurebakterium dem Subjekt oral verabreicht wird.

**Revendications**

**1.** Bactérie lactique isolée, qui est *Lactobacillus plantarum* sous-espèce *plantarum* PS128 et déposée auprès de DSMZ sous le numéro d'accès DSM 28632.

**2.** Composition comprenant la bactérie lactique isolée de la revendication 1, et un support.

**3.** Quantité efficace de la bactérie lactique isolée de la revendication 1, pour utilisation dans la prévention ou le traitement d'un trouble induit par le stress ou d'un trouble psychiatrique chez un sujet.

**4.** Quantité efficace de la bactérie lactique isolée de la revendication 1, pour utilisation dans un procédé de la revendication 3, dans laquelle le trouble induit par le stress ou le trouble psychiatrique est choisi dans le groupe constitué de l'anxiété, la dépression et le syndrome du côlon irritable.

**5.** Quantité efficace de la bactérie lactique isolée de la revendication 1, pour utilisation dans un procédé selon les revendications 3 et 4, dans laquelle après l'administration, un taux sérique de corticostérone est statistiquement significativement diminué chez le sujet.

**6.** Quantité efficace de la bactérie lactique isolée de la revendication 1, pour utilisation dans un procédé selon les revendications 3 et 4, dans laquelle après l'administration, un taux de neurotransmetteur est statistiquement significativement augmenté chez le sujet.

**7.** Quantité efficace de la bactérie lactique isolée de la revendication 1, pour utilisation dans un procédé selon la revendication 6, dans laquelle le neurotransmetteur est la dopamine.

8. Quantité efficace de la bactérie lactique isolée de la revendication 1, pour utilisation dans la prévention ou le traitement d'un trouble gastro-intestinal fonctionnel chez un sujet.

9. Quantité efficace de la bactérie lactique isolée de la revendication 1, pour utilisation dans la prévention ou le traitement d'une hypersensibilité viscérale chez un sujet.

10. Quantité efficace de la bactérie lactique isolée de la revendication 1, pour utilisation dans un procédé selon la revendication 9, dans laquelle l'hypersensibilité viscérale est associée à un trouble gastro-intestinal fonctionnel.

11. Quantité efficace de la bactérie lactique isolée de la revendication 1, pour utilisation dans un procédé selon les revendications 8 à 10, dans laquelle le trouble gastro-intestinal fonctionnel est choisi dans le groupe constitué d'une douleur abdominale fonctionnelle, du syndrome du côlon irritable, d'une constipation, d'une diarrhée fonctionnelle et d'une dyspepsie fonctionnelle.

12. Quantité efficace de la bactérie lactique isolée de la revendication 1, pour utilisation dans la prévention ou le traitement d'une douleur abdominale fonctionnelle, du syndrome du côlon irritable ou d'une constipation chez un sujet.

13. Quantité efficace de la bactérie lactique isolée de la revendication 1, pour utilisation dans un procédé selon les revendications 8 à 12, dans laquelle après l'administration, un taux d'un biomarqueur de sensation de douleur est statistiquement significativement diminué chez le sujet, facultativement dans lequel le biomarqueur est la substance P.

14. Quantité efficace de la bactérie lactique isolée de la revendication 1, pour utilisation dans un procédé selon les revendications 8 à 12, dans laquelle après l'administration, un signal d'électromyographie est statistiquement significativement diminué chez le sujet.

15. Quantité efficace de la bactérie lactique isolée de la revendication 1, pour utilisation dans un procédé selon les revendications 3 à 14, dans laquelle la bactérie lactique isolée est administrée par voie orale au sujet.

FIG. 1

FIG. 2

FIG. 3

FIG. 4

**A**

PS128 oral administration

| 0 day | 10 day | 24 day |
|---|---|---|
| Surgery for embedding electronod wire | CRD test | CRD test Sacrifice |

**B**

| -30 min | 0 min | 30 min |
|---|---|---|
| Colorectal distension (baseline test) | 5-HTP subcutaneous injection | Colorectal distension (hypersensitivity test) |

## FIG. 5

FIG. 6

FIG. 7

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 14259442 B

- US 14690502 B


**Non-patent literature cited in the description**

- **CHEN CL.** Visceral hypersensitivity in non-erosive reflux disease: neurogenic overwhelming in esophagus?. *Dig Dis Sci. Aug,* 2013, vol. 58 (8), 2131-2
- **COLLINS SM.** Translating symptoms into mechanisms: functional GI disorders. *Adv Physiol Educ. Dec,* 2007, vol. 31 (4), 329-31
- **DROSSMAN DA.** The functional gastrointestinal disorders and the Rome III process. *Gastroenterology,* April 2006, vol. 130 (5), 1377-90
- **KEOHANE J ; QUIGLEY EM.** Functional dyspepsia: the role of visceral hypersensitivity in its pathogenesis. *World J Gastroenterol.,* 07 May 2006, vol. 12 (17), 2672-6
- **MOSHIREE B ; ZHOU Q ; PRICE DD ; VERNE GN.** Central sensitisation in visceral pain disorders. *Gut,* 2006, vol. 55 (7), 905-8
- **BOUIN M ; PLOURDE V ; BOIVIN M ; RIBERDY M ; LUPIEN F ; LAGANIERE M ; VERRIER P ; POITRAS P.** Rectal distention testing in patients with irritable bowel syndrome: sensitivity, specificity, and predictive values of pain sensory thresholds. *Gastroenterology,* 2002, vol. 122, 1771-1777
- **CHIN J.** Alternation of substance P-containing neural pathways in a rat model of irritable bowel syndrome with rectal distension. *Dig Dis.,* 2006, vol. 7 (4), 211-8
- **NERI F ; CAVALLARI G ; TSIVIAN M ; BIANCHI E ; ALDINI R ; CEVENINI M ; GUIDETTI E ; PIRAS GL ; PARIALI M ; NARDO B.** Effect of colic vein ligature in rats with loperamide-induced constipation. *J Biomed Biotechnol.,* 2012, vol. 2012, 896162

- **FAUSTA SERAFINI ; FRANCESCA TURRONI ; PATRICIA RUAS-MADIEDO ; GABRIELE ANDREA LUGLI ; CHRISTIAN MILANI ; SABRINA DURANTI ; NICOLE ZAMBONI ; FRANCESCA BOTTACINI ; DOUWE VAN SINDEREN ; ABELARDO MARGOLLES.** Kefir fermented milk and kefiran promote growth of Bifidobacterium bifidum PRL2010 and modulate its gene expression. *International Journal of Food Microbiology,* 2014, vol. 178, 50-59
- **CHENG, F. C. ; J. S. KUO ; H. M. HUANG ; D. Y. YANG ; T. F. WU ; T. H. TSAI.** Determination of catecholamines in pheochromocytoma cell (PC-12) culture medium by microdialysis-microbore liquid chromatography. *J Chromatogr,* 2000, vol. A 870 (1-2), 405-411
- **CRYAN, J. F. ; A. DALVI ; S. H. JIN ; B. R. HIRSCH ; I. LUCKI ; S. A. THOMAS.** Use of dopamine-beta-hydroxylase-deficient mice to determine the role of norepinephrine in the mechanism of action of antidepressant drugs. *J Pharmacol Exp Ther,* 2001, vol. 298 (2), 651-657
- **DESBONNET, L. ; L. GARRETT ; G. CLARKE ; B. KIELY ; J. F. CRYAN ; T. G. DINAN.** Effects of the probiotic Bifidobacterium infantis in the maternal separation model of depression. *Neuroscience,* 2010, vol. 170 (4), 1179-1188
- **MILLSTEIN, R. A. ; A. HOLMES.** Effects of repeated maternal separation on anxiety- and depression-related phenotypes in different mouse strains. *Neurosci Biobehav Rev,* 2007, vol. 31 (1), 3-17